# EUROPEAN PATENT APPLICATION

(11) **EP 4 180 043 A1**
(43) Date of publication of application: **17.05.2023**
(21) Application number: 21842219.4
(22) Date of filing: 12.07.2021
(51) Int. Cl.: A61K 31/555, C07F 17/02, C07F 9/6581, A61P 35/00

(54) **RUTHENIUM COMPLEXES**

(30) Priority: 13.07.2020 ES 202030718
(71) Applicant: Universidad de Almeria, 04120 La Cañada de San Urbano (Almeria) (ES)
(72) Inventor: ROMEROSA NIEVAS, Antonio Manuel, 04120 Almería (ES); SCALAMBRA, Franco, 04120 Almería (ES); KORDESTANI MAHANI, Nazanin, 04120 Almería (ES)
(74) Representative: Tribalyte Ideas
(86) International application number: PCT/ES2021/070509
(87) International publication number: WO 2022/013467

(57) **Abstract**

The present invention relates to a new variety of ruthenium complexes, comprising a Ru derivative with p-cymene and dmoPTA or HdmoPTA. These complexes combine water solubility, high level of activity and specificity against different biomolecules, as well as air stability and easy administration. Additionally, the complexes exhibit biomolecule-specific activity, both in strength and activity, forming the basis for a range of drugs that can act specifically on certain targets and against the diseases in which they are implicated. The invention also relates to different processes for obtaining the aforementioned ruthenium complexes and to their use as medicaments or in the treatment of cancer.

## Description

### FIELD OF THE INVENTION

The present invention is comprised in the technical field of compounds with biological properties and, in particular, with anticancer activity. More specifically, the invention relates to ruthenium complexes, as well as to the process of obtaining same and uses thereof.

### BACKGROUND OF THE INVENTION

Cancer is one of the diseases that currently has the greatest impact on modern society, being the first cause of death in the western world. The number of drugs useful for the treatment of cancer is very limited, despite the efforts made in research to date. One of the known drugs with anticancer activity is cis-diamminedichloroplatinum(II), the properties of which were discovered by B. Rosenberg in the 1960s (see Rosenberg, B.; Van Camp, L.; Krigas, T.; Nature 1965, 205, 698). Both this compound and its derivative carboplatin are among the most widely used anticancer drugs in the clinical setting. However, despite their usefulness, platinum (Pt) complexes have serious disadvantages for the patient, due to their high toxicity, and they are also expensive to obtain. An alternative to Pt complexes are metallic compounds based on other metals, among which ruthenium (Ru) complexes stand out.

One of the first ruthenium compounds with +2 oxidation state (Ru(ll)) studied is cis-[Ru(bpy)₂(NH₃)₂]²⁺ (bpy = 2,2' bipyridine), analogous to cisplatin but without its high anticancer activity. Other examples are fac-[RuCl₃(NH₃)₂] (see Clarke, M.J.; Zhu, F.; Frasca, D.R. Chem. Rev. 1999, 99, 2511-2534; and Clarke, M.J. Met. Ions Biol. Syst. 1980, 11, 231-283), trans-[Hln][RuCl₄(Ind)₂] (Ind = indazole), merc-[Ru(terpy)Cl₃] (terpy = 2,2'-terpyridine), [Ru(chd-H₂)Cl₂] (chd = 1,2-cyclohexanediamine-tetraacetate), (see Keppler, B.K.; Henn, M.; Juhl, O.M.; Berger, M.R.; Niebl, R.; Wagner, F.E. Prog. Clin. Biochem. Med. 1989, 10, 41-69; Navakova, O.; Kasparova, J.; Vrana, O.; Van Vliet, P.M.; Reedijk, J.; Brabec, V. Biochemistry 1995, 34, 12369-2378; Vilaplana, R.A.; Gonzá/ez-Vílchez, F.; Gutiérrez-Puebla, E.; Ruíz-Valero, C. Inorg. Chim. Acta 1994, 224, 15-18; and Sava, G.; Gagliardi, R.; Bergamo, A.; Alessio, E.; Mestroni, G. Anticancer Res. 1999, 19, 969-972). Among the complexes of different metals studied, Ru(ll) complexes have proven to be more active than Ru(III) complexes, although other factors such as the water solubility of the complexes, or their partition coefficient (ratio between solubility in lipophilic and phobic environments), also affect their antiproliferative activity.

The administration and transport of these complexes through living organisms requires that their solubility in water be as high as possible. In this sense, the metallic complexes with the highest water solubility are those with hydrosoluble ligands. The best example of such compounds is trans-[RuCla(DMSO)Im][ImH] (NAMI-A), which has been the ruthenium compound known to have passed the largest number of clinical trials (see G. Sava, R.Gagliardi, A. Bergamo, E. Alessio, G. Mestroni, Anticancer Res. 1999, 19, 962-972; and M. Galanski, V. B. Arion, M. A. Jakupec, B. K. Keppler, Curr. Pharm. Des. 2003, 9, 2078-2089). Unlike cisplatin, NAMI-A does not inhibit growth of the primary tumor, but its main activity involves slowing the rate at which metastasis occurs (see G. Sava, S. Zorzet, C. Turrin, F. Vita, M. Soranzo, G. Zabucchi, M. Cocchietto, A. Bergamo, S. DiGiovine, G. Pezzoni, L. Sartor, S. Garbisa, Clin. Cancer Res. 2003, 9, 1898-1905*).*

Likewise, the different hydrophilic and lipophilic equilibria are essential for the *in vivo* behavior and efficacy of metallic complexes under physiological conditions. Thus, they must be sufficiently soluble in water (as it is the main component of physiological fluids), but also in organic media, to pass through the cell membrane. In this context, it has been observed that the combination of phosphane, arene, and aryl ligands contribute, not only to optimizing this type of equilibria in metallic complexes that are mainly in the 2nd and 3rd transition series, but also to regulating redox properties and steric factors (see C. Gaiddon, M.Pfeffer. Eur. J. Inorg. Chem. 2017, 1639-1654).

In the case of Ru(II), a family of RuCl-(η6-arene)(1κ*P*-PTA) type compounds, generically known as RAPTA, has been described. In addition to their low cytotoxicity compared to cisplatin, studies with commercial DNA (from calf thymus) suggest that RAPTA-C binds to the DNA molecule by binding to it and increasing the melting point of the DNA-RAPTA-C adduct. However, despite not showing selective binding against DNA *in vitro,* they are able to interact with histones as well as with RNA in the intracellular medium (see F. Scalambra, P. Lorenzo, I. de Ios Ríos, A. Romerosa. Eur. J. Inorg. Chem., 2019, 1529-1538*).*

Ru(ll) compounds bound to ligands such as PPh₃, PTA, and mPTA (see Antonio M. Romerosa, Tatiana Campos Malpartida, ChakerLidrissi, Mustapha Saoud, Manuel Serrano Ruiz, Mauricio Peruzzini, José Antonio Garrido Cárdenas, Federico Garcia Maroto Inorg. Chem. 2006, 45, 1289-1298; and Antonio Romerosa, Mustapha Saoud, Tatiana Campos-Malpartida, Chaker Lidrissi, Manuel Serrano-Ruiz, Maurizio Peruzzini, Jose Antonio Garrido-Cárdenas, Federico García-Maroto, Eur. J. Inorg. Chem. 2007, 2803-2812) have shown that they have good activity against DNA, being also very soluble in water, which is an additional advantage for their use as starting compounds for the synthesis of new drugs.

Although, as described, the progress in the improvement of ruthenium complexes against cancer has been considerable in recent years, there are still some limitations in them that it would be desirable to overcome. For example, there is still a need, in the present technical field, to obtain improved complexes that manage to stabilize the oxidation state of the Ru atom, providing at the same time an adequate hydrophilic/hydrophobic balance in such complexes. There is also, in the aforementioned field, a need to obtain Ru complexes that are stable, easy to prepare and possess high solubility in water and in organic solvents, as well as high lipid-solubility.

The present invention intends to meet said needs by means of novel ruthenium complexes, as well as of the processes for obtaining same and corresponding uses.

### BRIEF DESCRIPTION OF THE INVENTION

As described in the preceding section, a first object of the invention relates to a novel class of Ru complexes exhibiting high stability, hydrophilic/hydrophobic balance, ease of preparation, hydrosolubility, and lipid-solubility. Said object is realized by combining said complexes with ligands derived from 1,3,5-triaza-7-phosphaadamantane, known as PTA:

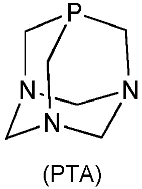

PTA is generally a ligand that provides stability and a good hydrophilic/hydrophobic balance suitable for the complex it forms. Also, the PTA derivatives 3,7-dimethyl-1,3,7-triaza-5-phosphabicyclo[3.3.1]nonane (referred to as dmoPTA), and 3,7-H-3,7-dimethyl-1,3,7-triaza-5-phosphabicyclo[3.3.1]nonane (HdmoPTA) are excellent ligands, which stabilize the +2 oxidation state on the Ru atom and are also easy to prepare and soluble in water and organic solvents. Both can also be easily subjected to protonation or deprotonation processes, respectively, depending on the desired level of lipid-solubility. Both ligands are shown below:

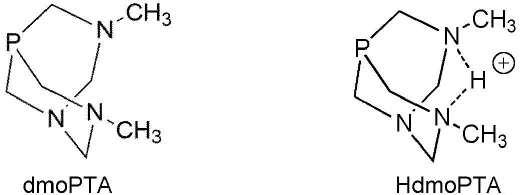

More specifically, the present invention proposes the use of Ru compounds with p-cymene and HdmoPTA or dmoPTA, lipid-soluble and/or hydrosoluble phosphines and halides, of general formulas I and II (see below) and exhibiting biological activity, in particular against cancer cells: 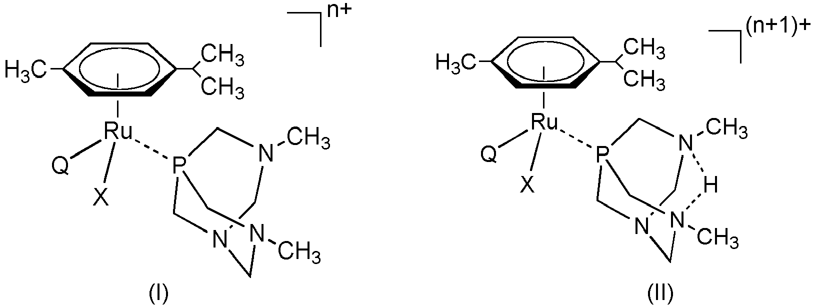 wherein Ru represents a ruthenium atom and Q, X represent halogens or lipid-soluble and/or hydrosoluble phosphines, charge n = 0, 1, 2, 3; including HdmoPTA and dmoPTA.

These complexes combine water solubility, high level of activity and specificity against different biomolecules, as well as air stability and easy administration. Advantageously, the biological activity of these compounds is higher or, at least, different from the biological activity of their parts. Also, due to the enormous variety of compounds that can be obtained by means of general formulas I and II, some of them can present specific activity against biomolecules, both in strenght and activity. These compounds thus form the basis of a range of drugs that can act on demand, specifically against certain target biomolecules and the diseases in which they are implicated.

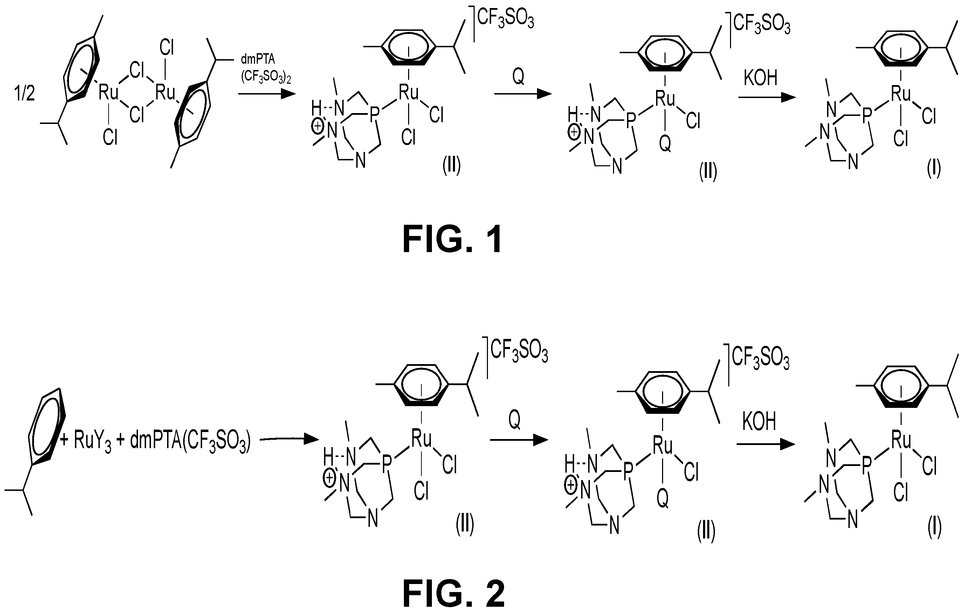

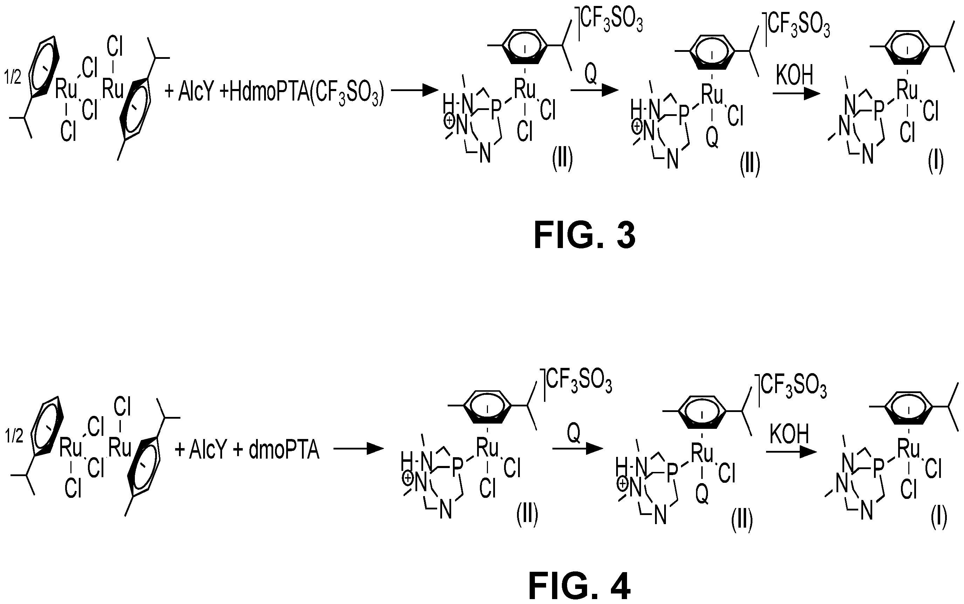

A second object of the present invention is a process for obtaining the ruthenium complexes of general formulas I and II. The processes start from either the ruthenium complex with p-cymene or from a ruthenium halogen. In the first case, substitution occurs with the dmPTA ligand, which opens during the reaction, giving rise to the HdmoPTA compound, or else the reaction with this ligand is started directly, but deprotonated. The combination of the different possibilities of obtaining the complexes gives rise to four different processes for obtaining these complexes:
a) Reaction of [Ru(p-cymene)Y₂]₂ (Y = halogen) complex with dmPTA (CF₃SO₃)₂ (dmPTA = dimethyl-adamantane phosphine), and subsequent reaction with phosphines (Q) soluble in water or in organic solvents (Figure 1). Type I complexes would be obtained from type II complexes by deprotonation with a strong or medium-strength base, such as NaOH, KOH, NH₃.
b) Direct reaction of a ruthenium halide (RuY₃; Y = F, Cl, Br, I) with p-cymene, with dmPTA and subsequent reaction with phosphines soluble in water or in organic solvents (Figure 2). Type I complexes would be obtained from type II complexes by deprotonation with a strong or medium-strength base, such as NaOH, KOH, NH₃.
c) Direct reaction of [Ru(p-cymene)Cl₂]₂ with an alkali halide (AlcY; Y = F, Cl, Br, I), with HdmoPTA(CF₃SO₃) (for II) and subsequent reaction with phosphines soluble in water or in organic solvents (Figure 3). Type I complexes would be obtained from type II complexes by deprotonation with a strong or medium-strength base, such as NaOH, KOH, NH₃.
d) Direct reaction of [Ru(p-cymene)Cl₂]₂ with an alkali halide, with dmoPTA (to obtain type I) and subsequent reaction with phosphines soluble in water or in organic solvents (Figure 4). Type II complexes are obtained by protonation of type I complexes with a strong or medium-strength acid, such as HCl, HCF₃SO₃, H₂SO₄, H₃PO₄, HClO₄, or CH₃CH₂COOH.

The above compounds, which are defined by general formulas I and II described above, combine increased anticancer properties with respect to their component parts, presenting a higher partition coefficient (solubility in water and lipids) than the parts of which they are composed and resistance to decomposition under air. The compounds have a better form of preparation, conservation, and administration, as well as a high biological activity accompanied by a high specificity against different tumors. Likewise, and as mentioned above, the specific composition of the complex determines its activity and selectivity, which, in addition, has a low toxicity against the rest of the cells of the organism, typical of Ru complexes.

Once the dmoPTA ligand is coordinated by the P atom to Ru, it can be coordinated again to another metal of a salt through the N atoms supporting the CH₃ groups. This coordination yields bis-heterometallic products, if the starting complex has only one dmoPTA (see scheme below, it is reacted with a bivalent metal halide-MY2), tris-heterometallic, if it has two dmoPTAs, and tetra-heterometallic, if it has three dmoPTAs.

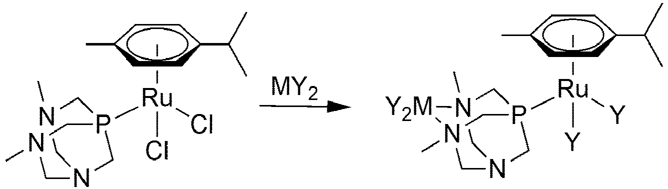

Furthermore, the obtained compounds can react through the metals bound to the CH₃N atoms to give rise to polyheterometallic systems.

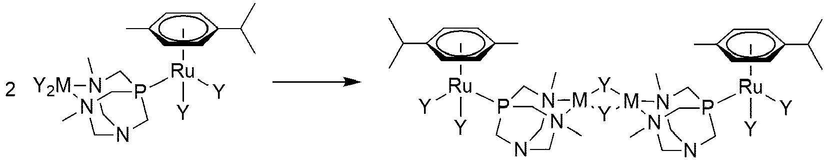

Other objects of the invention relate to monometallic, multi-metallic, and polymetallic ruthenium complexes according to any of the embodiments described herein, for use as a medicament or for use in the treatment of cancer.

The different objects of the invention are described overall in the claims attached hereto.

### DESCRIPTION OF THE DRAWINGS

Figure 1 shows a reaction of complex [Ru(p-cymene)Y₂]₂ (Y = halogen) with dmPTA (CF₃SO₃)₂ (dmPTA = dimethyl-adamantane phosphine) and a subsequent reaction with phosphines (Q) soluble in water or in organic solvents, according to a preferred embodiment of the invention. Type I complexes would be obtained from type II complexes by deprotonation with a strong or medium-strength base, such as NaOH, KOH, NH₃.
Figure 2 shows a direct reaction of a ruthenium halide (RuY₃; Y = F, Cl, Br, I) with p-cymene, with dmPTA and a subsequent reaction with phosphines soluble in water or in organic solvents, according to a preferred embodiment of the invention. Type I complexes would be obtained from type II complexes by deprotonation with a strong or medium-strength base, such as NaOH, KOH, NH₃.
Figure 3 shows a direct reaction of [Ru(p-cymene)Cl₂]₂ with an alkali halide (AlcY; Y = F, Cl, Br, I), with HdmoPTA(CF₃SO₃) (for II) and a subsequent reaction with phosphines soluble in water or in organic solvents, according to a preferred embodiment of the invention. Type I complexes would be obtained from type II complexes by deprotonation with a strong or medium-strength base, such as NaOH, KOH, NH₃.
Figure 4 shows a direct reaction of [Ru(p-cymene)Cl₂]₂ with an alkali halide, with dmoPTA (to obtain type I) and a subsequent reaction with phosphines soluble in water or in organic solvents, according to a preferred embodiment of the invention. Type II complexes are obtained by protonation of type I complexes with a strong or medium-strength acid, such as HCl, HCF₃SO₃, H₂SO₄, H₃PO₄, HClO₄, or CH₃CH₂COOH.

### DETAILED DESCRIPTION OF THE INVENTION

The first aspect of the invention relates to hydrosoluble ruthenium complexes, comprising dmoPTA (type I) and HdmoPTA (type II) derivatives, with p-cymene, halides, and hydrosoluble and/or lipid-soluble phosphines:

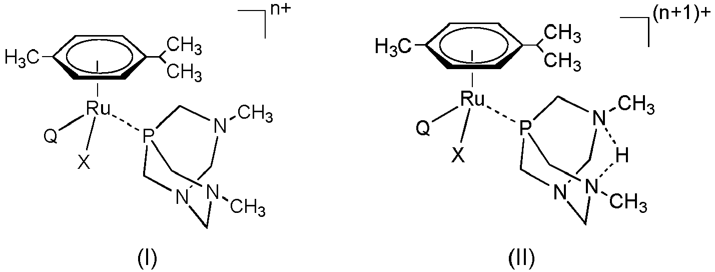

In the above formulas I and II, Ru represents a ruthenium atom; and Q, X represent, equally or separately: halogens, lipid-soluble or hydrosoluble phosphines; charge, n = 0, 1, 2, 3.

The ruthenium metal atom has a high biological activity. Moreover, it has been observed that its activity against biomolecules is enhanced when it is coordinated to the phosphine dmoPTA (type I complexes) and HdmoPTA (type II complexes). A preferred embodiment of the invention is considered any ruthenium complex of formula I and II in which Q and X represent the same or different ligand: F, Cl, Br, I; the lipid-soluble phosphines triphenylphosphine, trimethylphosphines, triethylphosphine, tripropylphosphine, tributylphosphine; hydrosoluble phosphines PTA (adamantane phosphine), dmPTA (dimethyl-adamantane phosphine), mPTA (methyl-adamantane phosphine) and phosphines with sulfonate, phosphate, carbonate, amine, ammonium, carboxylate, alcohol, aldehyde groups, and mixtures thereof. In this context, mixtures refer to phosphines possessing two or more of the above functional groups.

The second aspect of the invention relates to the different processes for obtaining the ruthenium complexes of general formulas I and II. In the processes described below, the components and substituents have the same meaning as that given above in describing the ruthenium complexes of general formulas I and II. The processes start from either the ruthenium complex with p-cymene or from a ruthenium halogen. In the first case, substitution occurs with the dmPTA ligand, which opens during the reaction, giving rise to the HdmoPTA compound, or else it is directly started from the reaction with that ligand, but deprotonated. The combination of the different possibilities of obtaining the complexes give rise to four main different processes for obtaining said complexes:
a) Reaction of [Ru(p-cymene)Y₂]₂ (Y = halogen) complex with dmPTA (CF₃SO₃)₂ (dmPTA = dimethyl-adamantane phosphine), and subsequent reaction with phosphines (Q) soluble in water or in organic solvents. Type I complexes would be obtained from type II complexes by deprotonation with a strong or medium-strength base, such as NaOH, KOH, NH₃ (Figure 1 of the present document).
b) Direct reaction of a ruthenium halide (RuY₃; Y = F, Cl, Br, I) with p-cymene, with dmPTA and subsequent reaction with phosphines soluble in water or in organic solvents. Type I complexes would be obtained from type II complexes by deprotonation with a strong or medium-strength base, such as NaOH, KOH, NH₃ (Figure 2 of the present document).
c) Direct reaction of [Ru(p-cymene)Cl₂]₂ with an alkali halide (AlcY; Y = F, Cl, Br, I), with HdmoPTA(CF₃SO₃) (for II) and subsequent reaction with phosphines soluble in water or in organic solvents. Type I complexes would be obtained from type II complexes by deprotonation with a strong or medium-strength base, such as NaOH, KOH, NH₃ (Figure 3 of the present document).
d) Direct reaction of [Ru(p-cymene)Cl₂]₂ with an alkali halide, with dmoPTA (to obtain type I) and subsequent reaction with phosphines soluble in water or in organic solvents. Type II complexes are obtained by protonation of type I complexes with a strong or medium-strength acid, such as HCl, HCF₃SO₃, H₂SO₄, H₃PO₄, HClO₄ or CH₃CH₂COOH (Figure 4 of the present document).

By way of illustration of the above processes, in a preferred embodiment of the invention the following steps would be performed:
i) Substituting a halogen of [Ru(p-cymene)Y₂]₂ (Y = F, Cl, Br, I) complex with dmPTA(CF₃SO₃)₂ to give rise to the type II complex [Ru(p-cymene)HdmoPTA(Y₂)]. The dmPTA ligand is transformed into HdmoPTA upon reaction with the solvent (MeOH, EtOH, and both linear and branched higher alcohols up to decanol). Type I complexes would be obtained from type II complexes by deprotonation with a strong or medium-strength base such as NaOH, KOH, NH₃ or other similar inorganic and organic bases.
ii) Reacting the complexes obtained in step i) above, once purified, with hydrosoluble and/or lipid-soluble phosphines.

In a preferred alternative embodiment of the invention, the following steps would be performed:
i) Direct reaction of a ruthenium salt (RuY₃) (Y = F, Cl, Br, I), with p-cymene and, subsequently, with dmPTA(CF₃SO₃)₂ to give rise to the type II complex [Ru(p-cymene)HdmoPTA(Y₂)]. Like in the above process, the type I compound is obtained by deprotonation of the compound of type II.
ii) The complexes obtained in the above step, once purified, are reacted with hydrosoluble and/or lipid-soluble phosphines.

In a preferred alternative embodiment of the invention, the following steps would be performed:
i) Type II complexes are obtained by reacting [Ru(p-cymene)Cl₂]₂ with an alkali halide (AlcY; Y = F, Cl, Br, I) and subsequently with HdmoPTA(CF₃SO₃). The type I complex is obtained at basic pH.
ii) Once the complexes obtained in the above step are purified, they are reacted with hydrosoluble and/or lipid-soluble phosphines.

In a preferred alternative embodiment of the invention, the reaction of [Ru(p-cymene)Cl₂]₂ with an alkali halide (AlcY; Y = F, Cl, Br, I) would be carried out with the dmoPTA ligand. The subsequent reaction with phosphines soluble in water or in organic solvents gives rise to the final type I complexes. Type II complexes are obtained upon acidification of type I complexes with acids strong, such as HCl, HCF₃SO₃, H₂SO₄, H₃PO₄, HClO₄, or medium-strength acids such as CH₃CH₂COOH.

All these processes are carried out in a reaction medium preferably comprising a solvent or mixture of solvents selected from water, ethanol, methanol, ethyl acetate, isopropanol, tert-butanol, ethylene glycol, diglyme (bis(2-methoxyethyl) ether), glyme (glycol dimethyl ether), chloroform, dichloromethane, benzene, toluene, acetone, tetrahydrofuran, dioxane, acetonitrile. Ethanol, acetone, and water, and mixtures thereof, are especially preferred.

The above processes can be carried out in temperature conditions of between -60°C and 150°C and between 0.5 and 100 atmospheres of pressure, with or without stirring. Preferably, the reactions are carried out in a temperature range of 5°C to 60°C and with pressures between 0.5 and 10 atmospheres.

The ruthenium salts (RuY₃) used in the context of the present invention can preferably be selected from soluble ruthenium halides.

Another aspect of the invention relates to a composition with biological activity comprising a ruthenium complex of general formulas I and/or II, combined with any additional compound with biological activity.

The following specific examples provided herein serve to illustrate the nature of the present invention. These examples are included solely for illustrative purposes and are not to be interpreted as limitations to the invention herein claimed.

### Example 1

Using the first process: Under a nitrogen atmosphere, [Ru(p-cymene)Cl₂]₂ (300 mg) is reacted in a volumetric flask with dmPTA(CF₃SO₃)₂ (dmPTA = dimethyl-1,3,5-triaza-7-phosphaadamantane) (376 mg) in 20 mL of EtOH. After 4 hours, under vigorous stirring at room temperature, 200 mg of PTA (adamantane phosphine) are added and the whole is refluxed for 2 hours. The obtained solution is filtered and by cooling the filtration water [Ru(p-cymene)Cl(HdmoPTA)(PTA)](CF₃SO₃)₂ complex is obtained as a precipitate which is isolated by filtration, washed with cold EtOH, and dried under vacuum.

### Example 2

Using the first process: [Ru(p-cymene)Cl(HdmoPTA)(PTA)](CF₃SO₃)₂ complex (300 mg) is dissolved in a mixture of 10 mL of EtOH/H₂O (1:1). KOH (30 mg) is added at room temperature. By evaporation of the solvent under vacuum to 5 mL the product [Ru(p-cymene)Cl(dmoPTA)(PTA)](CF₃SO₃)₂ precipitates and is filtered, washed with acetone, washed with ethyl ether, and dried under vacuum.

### Example 3

Using the second process: EtOH (30 mL) and, with vigorous stirring, RuCl₃ (200 mg), p-cymene (100 mg), and PTA (PTA = 1,3,5-triaza-7-phosphaadamantane) (100 mg) are added in a reaction balloon. The reaction mixture is refluxed for 4 hours, cooled, and evaporated to 5 mL. The solid formed is filtered, washed with acetone, washed with ethyl ether, and dried under vacuum. The solid is recrystallized in hot EtOH, giving rise to [Ru(p-cymene)Cl(HdmoPTA)(PTA)](CF₃SO₃)₂.

### Example 4

Using the third process: Chloroform (20 mL), [Ru(p-cymene)Cl₂]₂ (400 mg), LiCI (40 mg), and HdmoPTA(CF₃SO₃) (200 mg) are added in a 20 mm in diameter glass tube. After 4 hours of reflux, it is cooled and NaTPPMS (NaPPh₂PhSO₃) (600 mg) is added. The suspension is heated at 50°C for 3 hours, maintaining stirring and keeping the entire system under inert atmosphere. Upon cooling a product is formed that is filtered and air-dried. Said product is dissolved in 10 mL of EtOH/water (1:1) at 50°C. The obtained solution is cooled and evaporated under reduced pressure, obtaining [Ru(p-cymene)Cl(HdmoPTA)(TPPMS)](CF₃SO₃) complex.

### Example 5

Using the fourth process: [Ru(p-cymene)Cl₂]₂ (1000 mg), KBr (140 mg), and dmoPTA (400 mg) are added in a 100 mL balloon with 80 mL of a CHCl₃/EtOH (2:1) mixture. After4 hours under reflux, it is cooled and Na₃TPPTS (Na₃P(PhSO₃)₃) (2000 mg) and NaH (50 mg) are added, the mixture being heated under reflux. After 4 hours, it is cooled and the obtained suspension is filtered on a zeolite column 5 cm in height and 3 cm in diameter, which is washed with two portions of 2 mL of water. Na₂[Ru(p-cymene)Br(dmoPTA)(TPPTS)] complex is obtained by concentrating the resulting solution.

### Example 6

Using the fourth process: EtOH (40 mL), [Ru(p-cymene)Cl₂]₂ (300 mg), KI (70 mg), dmoPTA (100 mg), and PTA (110 mg) are added to a 250 mL balloon with vigorous stirring. The suspension is kept under reflux for 6 hours, maintaining stirring and under an inert atmosphere. The obtained solution is cooled and left to air-evaporate, obtaining [Ru(p-cymene)(I)(dmoPTA)(PTA)](I) complex.

Complexes with two HdmoPTA and dmoPTA ligands and three HdmoPTA and dmoPTA ligands would be obtained in a similar manner but changing the stoichiometry of the reagents.

### Example 7

According to the first general synthesis process: [Ru(p-cymene)Cl₂]₂ (300 mg) is reacted with dmPTA(CF₃SO₃)₂ (dmPTA = dimethyl-1,3,5-triaza-7-phosphaadamantane) (752 mg) in 30 mL of EtOH under an inert atmosphere. After 6 hours, 200 mg of PTA (adamantane phosphine) are added, and the whole is refluxed for 4 hours. The obtained solution is filtered, and [Ru(p-cymene)(HdmoPTA)₂(PTA)](CF₃SO₃)₄ complex is obtained by cooling the filtration water as a precipitate, that is isolated by filtration, washed with cold EtOH, and dried under vacuum.

### Example 8

Using the fourth general synthesis process: [Ru(p-cymene)Cl₂]₂ (1000 mg), KBr (140 mg), and dmoPTA (1200 mg) are added in a 100 mL balloon with 80 mL of a CHCl₃/EtOH (2:1) mixture. After 6 hours under reflux, the mixture is cooled with stirring. The obtained suspension is filtered on a synthesized glass plate, and the filtrate is washed with two portions of 10 mL of water. [Ru(p-cymene)(dmoPTA)₃]Cl₂ complex is obtained by concentrating the resulting solution.

The multimetallic compounds would be obtained from the complexes with one, two, or three HdmoPTA or dmoPTA, by reaction with metallic salts.

### Example 9

A suspension of [Ru(p-cymene)(HdmoPTA)₂(PTA)](CF₃SO₃)₄ (250 mg) is reacted with KOH (40 mg) and when the whole is completely dissolved, ZnCl₂ (100 mg) in EtOH is added and it is refluxed for 6 hours. [Ru(p-cymene){(dmoPTA-κ*P*,κ*N,N'*)-µ-ZnCl₂}₂(PTA)](CF₃SO₃)₂ in the form of microcrystals are obtained by cooling the resulting solution and are filtered under vacuum, washed with two portions of 10 mL of EtOH, and dried under vacuum.

### Example 10

[Ru(p-cymene)(dmoPTA)₃]Cl₂ complex (200 mg) suspended in 50 mL of MeOH is reacted with CoBr₂ (120 mg). The whole is refluxed for 6 hours and the resulting solution is left to slowly cool. The white powder obtained, [Ru(p-cymene){(dmoPTA-κ*P*,κ*N,N'*)₂-µ-ZnCl₂}₃]Cl₂ complex, is filtered under vacuum, washed with two portions of 10 mL of EtOH and 5 mL of Et₂O, and finally dried under reduced pressure.

The multimetallic complexes are obtained by means of reacting the previous complexes by reaction between them or with other metallic complexes.

### Example 11

A suspension of [Ru(p-cymene){(dmoPTA-κ*P*,κ*N,N*')-µ-ZnCl₂}₂(PTA)](CF₃SO₃) (150 mg) is reacted with AgCF₃SO₃ (30 mg) in 30 mL of MeOH. After 5 hours of stirring at room temperature the white-black precipitate formed is filtered and washed with three portions of 5 mL of EtOH at 50°C. The whole of the filtration water is concentrated to 2 mL and 5 mL of Et₂O are added. The precipitate formed is filtered and recrystallized in 20 mL of CHCl₃/acetone. The product [Ru(p-cymene)(PTA){(dmoPTA-κ*P*,κ*N,N'*)₂-µ-ZnCl}₂-µ-{ClZn-µ-(dmoPTA-κ*P*,κ*N,N*')}₂(PTA)(p-cymene)Ru](CF₃SO₃)₄ precipitates as a microcrystalline powder, which is filtered on a porous glass plate, washed with two portions of 5 mL of EtOH at 5°C, and dried under vacuum.

### Example of anticancer properties of compounds of this type

As an illustrative example of the anticancer properties of the compounds of the invention, [Ru(η⁶-C₁₀H₁₄)(Cl₂)(HdmoPTA)](OSO₂CF₃) complex showed antiproliferative activity indicated by the cell survival half-life factor (IC50) of 32.09±0.30 µM versus that of cisplatin which, in the same experiment, was 45.6±8.08 µM. Three examples of bi-heterometallic compounds which would be obtained from that compound present better activity as shown by their IC50: IC50 Ru-Zn: 9.07±0.27; IC50 Ru-Co: 5.40±0.19; IC50 Ru-Ni: 7.15±0.30 µM. Ru-Zn = [Ru(η⁶-C₁₀H₁₄)(Cl₂)-µ-dmoPTA-1κ*P*:2κ²*N,N'*-ZnCl₂]. Ru-Co = [Ru(η⁶-C₁₀H₁₄)(Cl₂)-µ-dmoPTA-1κ*P*:2κ²*N,N'*-CoCl₂]. Ru-Ni = [Ru(η⁶-C₁₀H₁₄)(Cl₂)-µ-dmoPTA-1κ*P*:2κ²*N,N'-*NiCl₂].

## Claims

1. A ruthenium complex, **characterized in that** it comprises a Ru derivative with p-cymene and dmoPTA, according to formula (I): 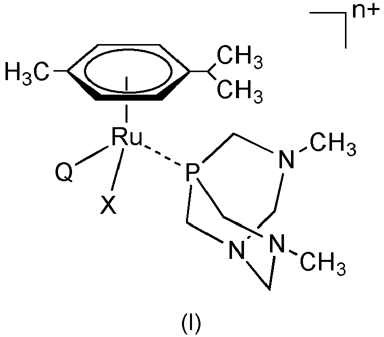 wherein:
- Ru represents a ruthenium atom;
- Q and X, which are the same as or different from one another and represent a halogen or a lipid-soluble or hydrosoluble phosphine;
- n = 0, 1, 2, 3; it represents the charge of the complex.

2. The ruthenium complex according to the preceding claim, wherein dmoPTA is protonated, forming HdmoPTA, according to formula (II):

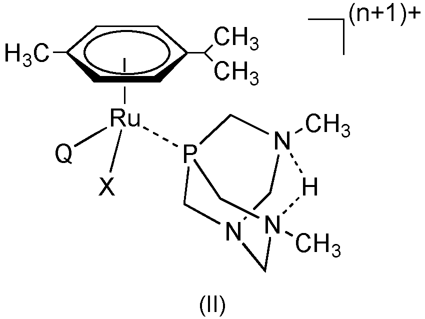

3. The ruthenium complex according to any of the preceding claims, wherein Q and/or X represent:
- a triphenylphosphine, tribenzylphosphine, triethylphosphine, and tripropylphosphine;
- F, Cl, Br or I;
- a hydrosoluble phosphine, selected from phosphines of sulfonate, phosphate, carbonate, amine, ammonium, carboxylate, alcohol, aldehyde groups or mixtures thereof, adamantane phosphine (PTA) or derivatives thereof;
with Q and X being the same as or different from one another.

4. A process of obtaining a ruthenium complex according to claim 1, **characterized in that** it comprises performing the following steps:
i) reacting [Ru(p-cymene)Cl₂]₂ with an alkali halide AlcY, wherein Y = F, Cl, Br, I, with dmoPTA;
ii) substituting one or two metal-coordinated halides with a phosphine soluble in water and/or in organic solvents.

5. The process of obtaining a ruthenium complex according to claim 2, **characterized in that** it comprises performing the following steps:
i) substituting a halide in [Ru(p-cymene)Y₂]₂(CF₃SO₃), wherein Y = F, Cl, Br, I, with a dmPTA ligand which, once coordinated, is transformed into the HdmoPTA ligand;
ii) substituting one or two metal-coordinated halides with a phosphine soluble in water and/or in organic solvents.

6. The process of obtaining a ruthenium complex according to claim 2, **characterized in that** it comprises performing the following steps:
i) directly reacting a ruthenium salt RuY₃, where Y = F, Cl, Br, I, with p-cymene and dmPTA which, once coordinated, is transformed into the HdmoPTA ligand;
ii) substituting one or two metal-coordinated halides with a phosphine soluble in water and/or in organic solvents.

7. The process of obtaining a ruthenium complex according to claim 2, **characterized in that** it comprises performing the following steps:
i) reacting [Ru(p-cymene)Cl₂]₂ with an alkali halide AlcY, wherein Y = F, Cl, Br, I, and HdmoPTA(CF₃SO₃);
ii) substituting one or two metal-coordinated halides with a phosphine soluble in water and/or in organic solvents.

8. The process for obtaining a ruthenium complex according to claim 1 by deprotonation with a base of a complex according to claim 2.

9. The process for obtaining a ruthenium complex according to claim 2 by protonation with an acid of a complex according to claim 1.

10. A process for obtaining multimetallic Ru complexes from the complexes obtained by the preceding claims, by reacting said complexes with transition metal salts with halides, sulfate, nitrate, nitrite, phosphate, thiocyanate, and cyanide, acetylacetonate and amines.

11. The process for obtaining polymetallic complexes from the complexes obtained by the preceding claims, by reacting said complexes with silver salts and transition metal salts with halides, sulfate, nitrate, nitrite, phosphate, thiocyanate, and cyanide, acetylacetonate and amines.

12. The process according to any of claims 4-11 comprising the use of one or more solvents comprising water, ethanol, methanol, ethyl acetate, isopropanol, tert-butanol, ethylene glycol, diglyme (bis(2-methoxyethyl) ether), glyme (glycol dimethyl ether), chloroform, dichloromethane, benzene, toluene, acetone, tetrahydrofuran, dioxane and/or acetonitrile, and/or mixtures thereof.

13. The process according to any of claims 4-12, wherein at least one of its steps is partially or completely carried out in a temperature range comprised between -60°C and 150°C with or without stirring, and pressure comprised between 0.5 and 10 atm.

14. The process according to any of claims 4-13, comprising the use of ruthenium salts RuY₃, wherein Y = F, Cl, Br, I, for the synthesis of the ruthenium complex and/or for the synthesis of the p-cymene complex.

15. The process according to any of claims 4-14, comprising the use of alkali halide salts ranging from fluorides to iodides.

16. A ruthenium complex according to any of the preceding claims for use as a medicament

17. A ruthenium complex according to any of the preceding claims for use in the treatment of cancer.
